# EUROPEAN PATENT APPLICATION

(11) **EP 2 985 055 A1**
(43) Date of publication of application: **17.02.2016**
(21) Application number: 15180304.6
(22) Date of filing: 07.08.2015
(51) Int. Cl.: A61N 1/378, A61N 1/39, H01G 4/015

(54) **CAPACITOR CONFIGURATION FOR SUBCUTANEOUS IMPLANTABLE CARDIOVERTER-DEFIBRILLATORS**

(30) Priority: 08.08.2014 US 201462034804 P; 06.08.2015 US 201562201595 P; 06.08.2015 US 201562201609 P
(71) Applicant: Newpace Ltd., 3088900 Caesarea (IL)
(72) Inventor: BRODER, Avi, 4971202 Petach Tikva (IL); MOCHA, Moty, 5020000 Beit Dagan (IL); STROMMER, Gera M., 3475950 Haifa (IL)
(74) Representative: Beck Greener

(57) **Abstract**

Capacitor configuration for use in a subcutaneous implantable cardioverter-defibrillator having a tubular shape, including a first plurality of high volumetric efficiency capacitors, each one of the first plurality of high volumetric efficiency capacitors having a disc-shape, the first plurality of high volumetric efficiency capacitors being coupled in parallel and arranged in a stack as a first array of capacitors, thereby forming a cylindrically-shaped capacitor.

## Description

The disclosed technique relates to implantable cardioverter-defibrillators, in general, and to capacitor configurations and arrangements for flexible subcutaneous implantable cardioverter-defibrillators, in particular.

An implantable cardioverter-defibrillator (herein abbreviated ICD) is a medical device combining a cardioverter and a defibrillator into a single implantable unit. An ICD is thus a small battery-powered electrical impulse generator that is implanted in patients who are at risk of sudden cardiac death due to ventricular fibrillation (which can be remedied via defibrillation) and ventricular tachycardia (which can be remedied via cardioversion). As both defibrillation and cardioversion require significant amounts of energy to be delivered to the heart in order to be effective, an ICD usually requires a plurality of high energy capacitors in order to store sufficient energy per electrical impulse to be delivered by the ICD to a patient suffering from ventricular fibrillation (herein abbreviated VF) or ventricular tachycardia (herein abbreviated VT).

One kind of known ICD is implanted intravascularly or intracardially, having leads positioned within the heart as well as being attached to the outer surface of the heart. The leads are substantially electrodes capable of providing high voltage electrical impulses to the heart. Such ICDs require the plurality of high energy capacitors to provide about 35-40 joules of energy per electrical impulse, as well as having a high voltage per electrical impulse in the range of 700-800 volts to properly defibrillate, for example, a patient suffering from VF. Another kind of known ICD is implanted subcutaneously and includes leads that are placed under the skin and without direct contact with the heart. Such ICDs are also known in the art as subcutaneous ICDs. The electrical impulse in a subcutaneous ICD needs to pass through muscles, the lungs and bones to defibrillate the heart as the leads are placed subcutaneously and are not in contact with the heart. As such, subcutaneous ICDs must employ even higher energy and voltage levels than intracardiac ICDs. For example, the only currently marketed available subcutaneous ICD, produced by Boston Scientific and sold under the brand name Emblem™ S-ICD System, generates approximately 80 joules and 1000 volts per electrical impulse. Either way however, both an intracardiac ICD and a subcutaneous ICD require a plurality of high energy capacitors that can store the requisite levels of energy and can support the required voltages per electrical impulse.

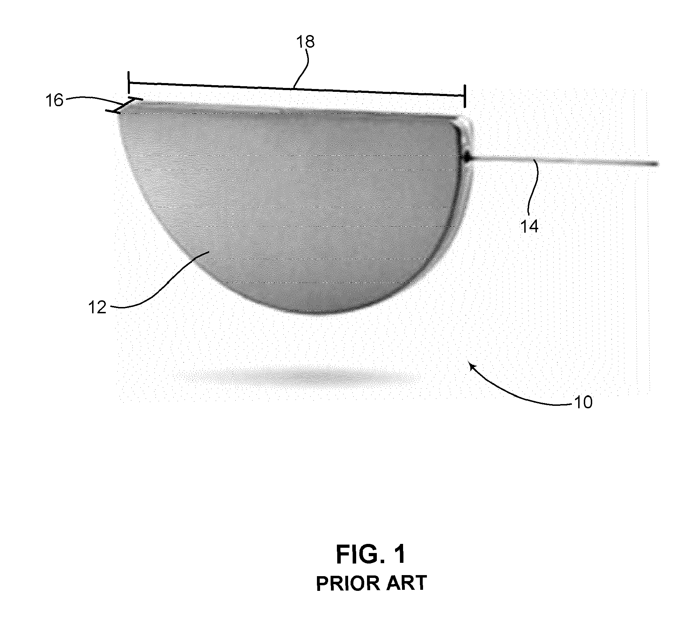

Wet tantalum capacitors are used in most state-of-the-art ICDs. Such capacitors are used due to their ability to store high energy levels as well as their ability to maintain high voltages, and thus have a high volumetric efficiency. Most ICDs, including the Emblem™ S-ICD System, have a can and leads design. In such ICDs the capacitors are stored in the can and are therefore shaped, designed and built to accommodate the shape of the can. The most common capacitor shape used in ICDs is in the form of a half circle or a shape similar or close to a half circle, also known as a 'D-shape'. Reference is now made to Figure 1, which is a schematic illustration of a wet tantalum capacitor, generally referenced 10, as known in the prior art. Wet tantalum capacitor 10 includes a 'D-shape' body 12 and a connector 14 for electrically coupling wet tantalum capacitor 10 with other electronic components (not shown). As shown, a thickness 16 of 'D-shape' body 12 is substantially smaller in comparison to a length 18 of 'D-shape' body 12, giving 'D-shape' body 12 a substantially flat appearance. Wet tantalum capacitor 10 is employed and used in state-of-the-art ICDs and subcutaneous ICDs.

One of the challenges in building capacitors for ICDs and subcutaneous ICDs is energy efficiency per volume. On the one hand, substantial amounts of energy are required for ICDs, and even more so for subcutaneous ICDs to function effectively. There is thus a desire to use larger sized capacitors to meet these requirements. However in contradistinction, since ICDs and subcutaneous ICDs are implanted in a patient, there is a desire to make such devices as small and as unobtrusive as possible. All currently marketed ICDs use capacitors to store and provide energy for electrical impulses, such as wet tantalum capacitor 10. Such capacitors enable an appropriate energy efficiency per volume to be achieved while also meeting both the energy and voltage requirements of ICDs and subcutaneous ICDs, together with the shape and volume constraints of such devices. Capacitor shapes and designs for ICDs and subcutaneous ICDs are known in the art and are described in the following patents: US 5,749,911 to Westlund, US 7,715,174 to Beauvais et al. and US 7,656,646 to Sherwood.

The configuration, shape and design of wet tantalum capacitor 10 as shown in Figure 1 however is not suitable for a flexible shaped ICDs such as the implantable subcutaneous string heart device (herein abbreviated ISSD), being developed by NewPace Ltd., due to the low ratio between capacitance and size when taking the spatial configuration constraints of the ISSD into account. The ISSD being developed by NewPace Ltd. is a tubular or cylindrical structure having a flexible shape which is designed to be implanted subcutaneously. The ISSD may have a circular cross-section and may also have an elliptical cross-section. A cylindrical-shaped capacitor, having a cylindrical-shaped anode (e.g., one made from tantalum) and a cathode (e.g., a foil covering the anode) would thus seem to be an ideal candidate for the ISSD. However, one of the problems in building and using cylindrical-shaped wet tantalum capacitors for the ISSD is that a cylindrical-shaped anode increases the overall distance between any point in the anode to any point in the cathode. This increase in distance causes an increase in the inner resistance of such a capacitor, thereby increasing the equivalent series resistance (herein abbreviated ESR) for such capacitors. The increase in ESR would thus not enable sufficient energy to be stored and delivered to the heart of a patient subcutaneously given the size and volume constraints of the ISSD. As an example, an intravascular heart device which was being developed by InnerPulse Inc., was designed to be implanted within the vascular system of the heart and would use a cylindrical-shaped capacitor to provide electrical impulses, as outlined in US Patent Application Publication No. 2012/0123489 A1 to Ransbury et al. The energy requirements of the InnerPulse Inc. device are similar to the energy requirements of an ICD. However the cylindrical-shaped capacitor of the InnerPulse Inc. device provides substantially less energy than required by a subcutaneous ICD, thus not making such a capacitor design and configuration suitable for the ISSD. A cylindrical-shaped capacitor with a higher capacitance meeting the energy requirements for a flexible subcutaneous ICD, such as the ISSD of NewPace Ltd., is challenging due to the shape and volume constraints of the ISSD. It is known in the art that high performance capacitors can exhibit a maximum stored energy of about 7 joules per cubic centimeter, storing a voltage of about 250 volts per capacitor. Hence, any ICD employing high performance capacitors requires the connection of several high performance capacitors in series in order to get to the required levels of voltage and energy for an intravascular ICD. This is all the more so in the case of a flexible subcutaneous ICD which has even higher voltage and energy requirements, such as for example 1200 volts and 70 joules, as mentioned above.

The disclosed technique overcomes the disadvantages of the prior art by providing a novel capacitor arrangement and configuration for use in a subcutaneous ICD.

According to an embodiment of the disclosed technique, there is thus provided a capacitor configuration for use in a subcutaneous implantable cardioverter-defibrillator having a tubular shape. The capacitor configuration comprises a first plurality of high volumetric efficiency capacitors. Each one of the high volumetric efficiency capacitors has a disc-shape. The high volumetric efficiency capacitors are coupled in parallel and are arranged in a stack as a first array of capacitors, thereby forming a cylindrically-shaped capacitor.

According to a further embodiment of the disclosed technique, the capacitor configuration further comprises a second plurality of high volumetric efficiency capacitors. The second plurality of high volumetric efficiency capacitors are coupled in parallel and are arranged in a stack as a second array of capacitors. The first array of capacitors is coupled in series with the second array of capacitors.

According to a first aspect of the present invention, there is provided a capacitor configuration for use in a subcutaneous implantable cardioverter-defibrillator (ICD) having a tubular shape, characterized by:said capacitor configuration comprising a first plurality of high volumetric efficiency capacitors, each one of said first plurality of high volumetric efficiency capacitors having a disc-shape, said first plurality of high volumetric efficiency capacitors being coupled in parallel and arranged in a stack as a first array of capacitors, thereby forming a cylindrically-shaped capacitor.

In one embodiment, said first plurality of high volumetric efficiency capacitors are wet tantalum capacitors.

In one embodiment, said disc-shape has a circular cross-section.

In one embodiment, said disc-shape has an elliptical cross-section.

In one embodiment, said first array of capacitors arranged in said stack minimizes equivalent series resistance (ESR) in said cylindrically-shaped capacitor.

In one embodiment, said first array of capacitors enables high energy and high voltage storage.

In one embodiment, the capcitor configuration further comprises a second plurality of high volumetric efficiency capacitors, said second plurality of high volumetric efficiency capacitors being coupled in parallel and arranged in a stack as a second array of capacitors, wherein said first array of capacitors is coupled in series with said second array of capacitors.

In one embodiment,said first array of capacitors and said second array of capacitors together enable high energy and high voltage storage.

In one embodiment, said high energy is at least 50 joules and said high voltage is at least 1000 volts.

In one embodiment, the capacitor configuration further comprises a cover for said first array of capacitors.

In one embodiment, said cover provides at least one of mechanical support and protection to said first array of capacitors.

In one embodiment, said first array of capacitors and said second array of capacitors enable flexibility in said subcutaneous ICD having said tubular shape.

Embodiments of the present invention will now be described in detail with reference to the accompanying drawings, in which:
Figure 1 is a schematic illustration of a wet tantalum capacitor, as known in the prior art;
Figure 2 is a schematic illustration of a high energy capacitor configuration for use in a flexible subcutaneous ICD, constructed and operative in accordance with an embodiment of the disclosed technique;
Figure 3 is a schematic illustration of a single coin-shaped wet tantalum capacitor, constructed and operative in accordance with another embodiment of the disclosed technique;
Figure 4 is a schematic illustration of a plurality of the coin-shaped wet tantalum capacitors of Figure 3, configured as a capacitor array, constructed and operative in accordance with a further embodiment of the disclosed technique;
Figure 5 is a schematic illustration of various capacitor configurations, constructed and operative in accordance with another embodiment of the disclosed technique;
Figure 6 is a schematic illustration of the capacitor array of Figure 4 including a cover, constructed and operative in accordance with a further embodiment of the disclosed technique; and
Figure 7 is a schematic illustration of a high energy wet tantalum capacitor configuration for use in a flexible subcutaneous ICD, constructed an operative in accordance with another embodiment of the disclosed technique.

The disclosed technique overcomes the disadvantages of the prior art by providing a novel high energy capacitor configuration for a flexible subcutaneous ICD that achieves the energy and voltage requirements for a subcutaneous ICD while also conforming to volume and shape constraints of a tubular shaped device for subcutaneous implantation. The novel capacitor configuration is also suitable for an implantable subcutaneous string heart device as mentioned above. The high energy capacitor configuration can further be used for a wet tantalum capacitor configuration exhibiting a cylindrical shape suitable to be inserted into a flexible tubular structure such as the ISSD of NewPace Ltd. The ISSD is a flexible and unitary subcutaneous ICD which is implanted under the skin of a patient and delivers electrical impulses with characteristics similar to a subcutaneous ICD. As mentioned above, the ISSD is also a flexible and tubular structure, which requires higher energy and voltage per electrical impulse as compared to intravascular and intracardiac ICDs, since the ISSD is a subcutaneous ICD and the electrical impulse energy needs to cross more tissue, muscles and bone before reaching the heart.

According to the disclosed technique, a wet tantalum capacitor is formed in the shape of a disc, coin or ellipse, conforming to the volume and shape of the ISSD lengthwise. The disc or coin shape may be circular in cross-section or elliptical in cross-section. A plurality of capacitors can thus be coupled in parallel, forming a capacitor array which conforms to the volume and shape of the ISSD lengthwise. A plurality of capacitor arrays can be coupled in series giving a capacitor configuration, thereby delivering sufficient energy and voltage per electrical impulse while conforming to the shape and volume of the ISSD lengthwise. As each capacitor array is coupled with another capacitor array, a degree of flexibility is enabled between capacitor arrays. The capacitor configuration according to the disclosed technique is capable of delivering the requisite amounts of voltage and energy to sufficiently defibrillate the heart of a patient with leads that are located under the skin and not within the heart or the vascular system. Furthermore, such a capacitor configuration may be integrated into a tubular and flexible device with a limited outer diameter and length for each capacitor.

In general, it is a challenge to squeeze high energy capacitors, such as wet tantalum capacitors, into a small cylindrical shape, as required for a flexible and tubular device such as the ISSD of NewPace Ltd. As described above, most prior art ICDs and even prior art subcutaneous ICDs utilize flat, semi-circle or 'D-shaped' capacitors to meet energy and voltage requirements while also satisfying the spatial requirements of such devices. According to the disclosed technique, due to the round, elliptical shape of the ISSD of NewPace Ltd., a novel capacitor configuration was required which needed to have a generally cylindrical shape to match the shape of the ISSD of NewPace Ltd. while also providing sufficient energy and voltage as required for a subcutaneous ICD, which as mentioned above is about 70-80 joules and around 1100-1200 volts. It is noted that the energy and voltage requirements for a subcutaneous ICD such as the ISSD may be slightly lower, such as 50 joules and 1000 volts, however these requirements are nonetheless higher than the requirements for an intracardiac ICD.

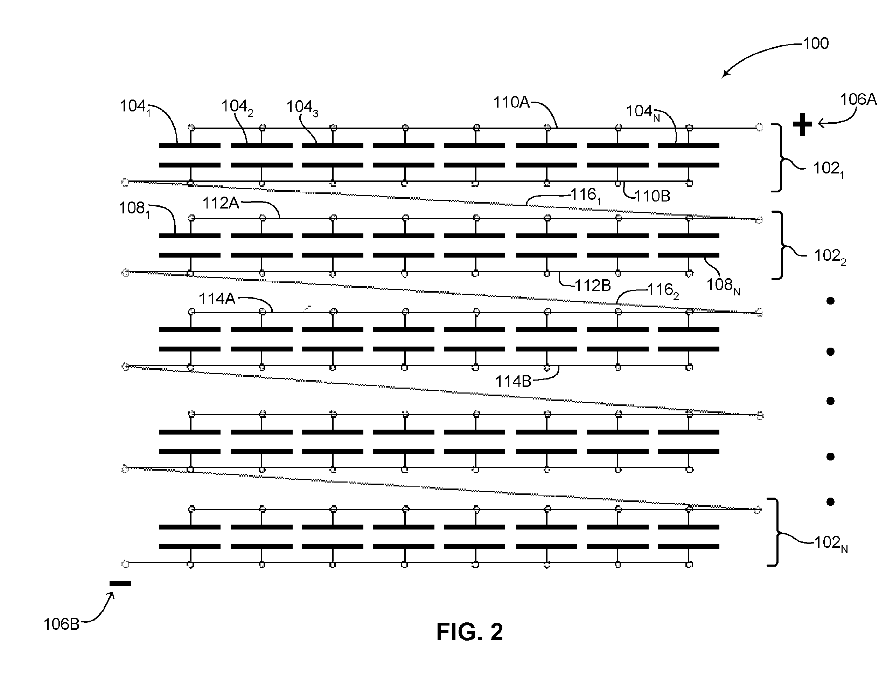

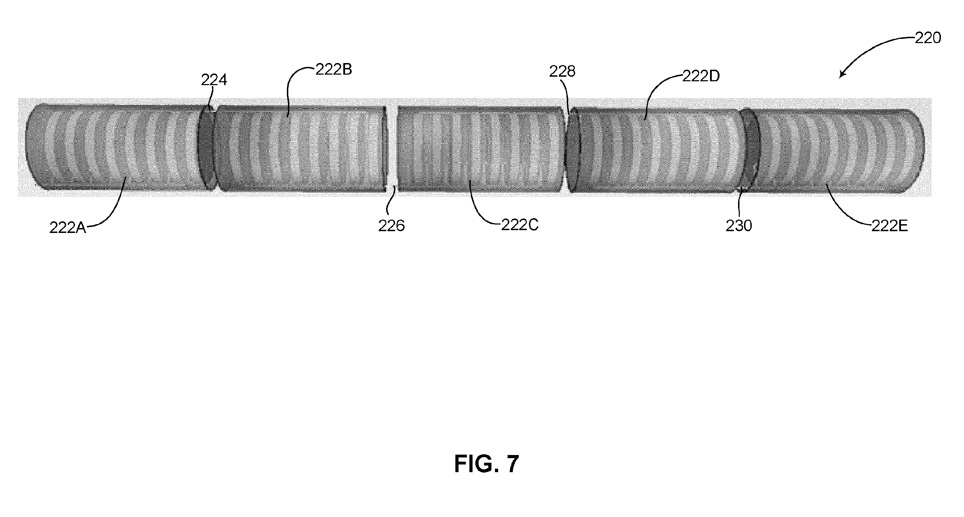

Reference is now made to Figure 2 which is a schematic illustration of a high energy capacitor configuration for use in a flexible subcutaneous ICD, generally referenced 100, constructed and operative in accordance with an embodiment of the disclosed technique. High energy capacitor configuration 100 is more of a schematic electrical diagram and does not represent the actual physical placement of individual capacitors according to the disclosed technique. The physical placement of individual capacitors is shown and explained below in Figure 7. What is shown in Figure 2 is how a plurality of high energy capacitors are to be coupled in order to achieve the energy and voltage requirements of a subcutaneous ICD.

According to one embodiment of the disclosed technique, a high energy capacitor configuration includes a plurality (i.e., two or more) of flat wet tantalum capacitors, each capacitor being in the shape of a coin or disc. A plurality of capacitors is coupled in parallel, thereby creating an array of capacitors. Each array is then coupled in series with another array, thereby accumulating the required energy and voltage for a subcutaneous ICD. As shown in Figure 2, high energy capacitor configuration 100 includes a plurality of capacitor arrays 102₁-102_{N} coupled in series. Each one of capacitor arrays 102₁-102_{N} includes a plurality of capacitors coupled in parallel. For example, capacitor array 102₁ includes a plurality of capacitors 104₁-104_{N} coupled in parallel. Capacitor array 102₂ includes a plurality of capacitors 108₁-108_{N} coupled in parallel, and so on. As shown, a set of electrical lines 110A and 110B couple plurality of capacitors 104₁-104_{N} in parallel. A set of electrical lines 112A and 112B couple plurality of capacitors 108₁-108_{N} in parallel. A set of electrical lines 114A and 114B coupled the next plurality of capacitors in parallel, and so on. As an example, Figure 2 depicts five capacitors arrays, with each capacitor array including eight capacitors. As shown, one end of high energy capacitor configuration 100 forms an anode 106A whereas the other end of high energy capacitor configuration 100 forms a cathode 106B. Also shown is the serial coupling between each capacitor array. As shown, capacitor 104₁ of capacitor array 102₁ is coupled in series with capacitor 108_{N} of capacitor array 102₂ via an electrical line 116₁. This is also shown for the next capacitor array via an electrical line 116₂, and so on.

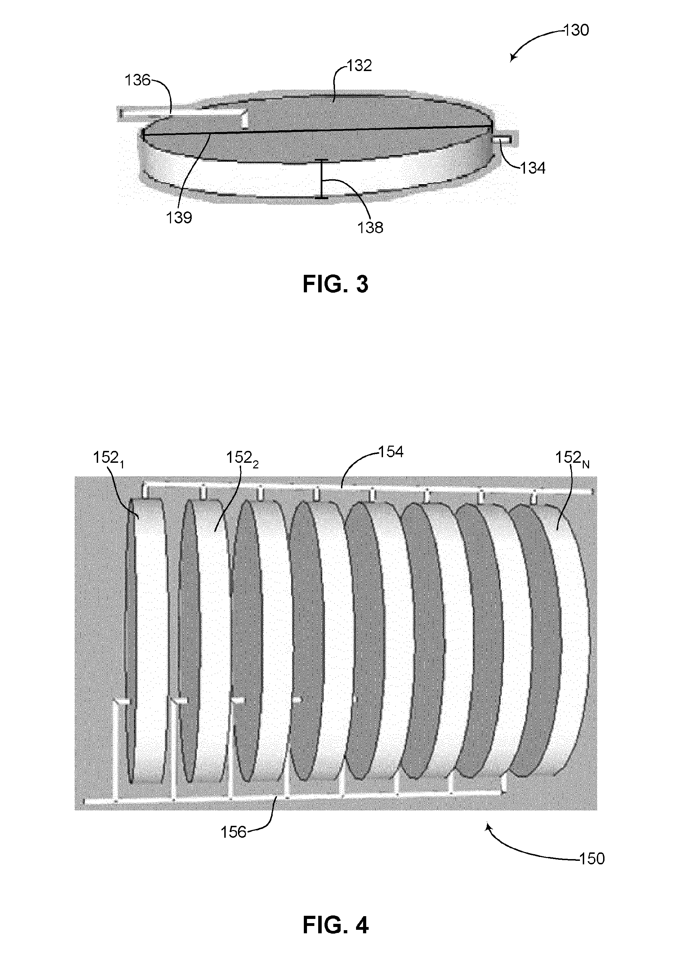

Reference is now made to Figure 3 which is a schematic illustration of a single coin-shaped wet tantalum capacitor, generally referenced 130, constructed and operative in accordance with another embodiment of the disclosed technique. Shown in Figure 3 is a capacitor body 132 which has a coin-shape or disc-shape. Capacitor body 132 is shown having a coin-shape or disc-shape with a cross--section that is substantially circular. According to the disclosed technique, the capacitor body having a coin-shape or disc-shape can also have a cross-section which is substantially elliptical. Such a disc-shape with an elliptical cross-section could be used in an ISSD or other flexible subcutaneous implantable device that has an elliptical cross-section. Capacitor body 132 may be a wet tantalum capacitor. Capacitor body 132 includes a first electrical coupler 134 and a second electrical coupler 136. First electrical coupler 134 and second electrical coupler 136 may be respectively an anode and a cathode of capacitor body 132 or vice-versa. As shown in Figure 4 below, first and second electrical couplers 134 and 136 can be used to couple a plurality of capacitor bodies together. Capacitor body 132 has a thickness 138 which is substantially smaller than a diameter 139 of capacitor body 132. Capacitor body 132 is thus shaped like a flat disc or coin.

Reference is now made to Figure 4 which is a schematic illustration of a plurality of the coin-shaped wet tantalum capacitors of Figure 3, configured as a capacitor array, generally referenced 150, constructed and operative in accordance with a further embodiment of the disclosed technique. Capacitor array 150 includes a plurality of coin-shaped high energy capacitors 152₁, 152₂ and 152_{N}. As shown, an electrical line 154 couples one side (for example, the anode) of the plurality of coin-shaped high energy capacitors in parallel, whereas an electrical line 156 couples the other side (for example, the cathode) of the plurality of coin-shaped high energy capacitors in parallel. Electrical lines 154 and 156 are substantially similar to set of electrical lines 110A and 110B (Figure 2). As shown, capacitor array 150 includes plurality of coin-shaped high energy capacitors 152₁, 152₂ and 152_{N} positioned in a stack formation, giving capacitor array 150 an overall cylindrical shape being formed of individual thin and flat disc-shaped capacitors. By coupling a plurality of high energy capacitors together in parallel, a sufficiently high energy and voltage per capacitor array can be achieved.

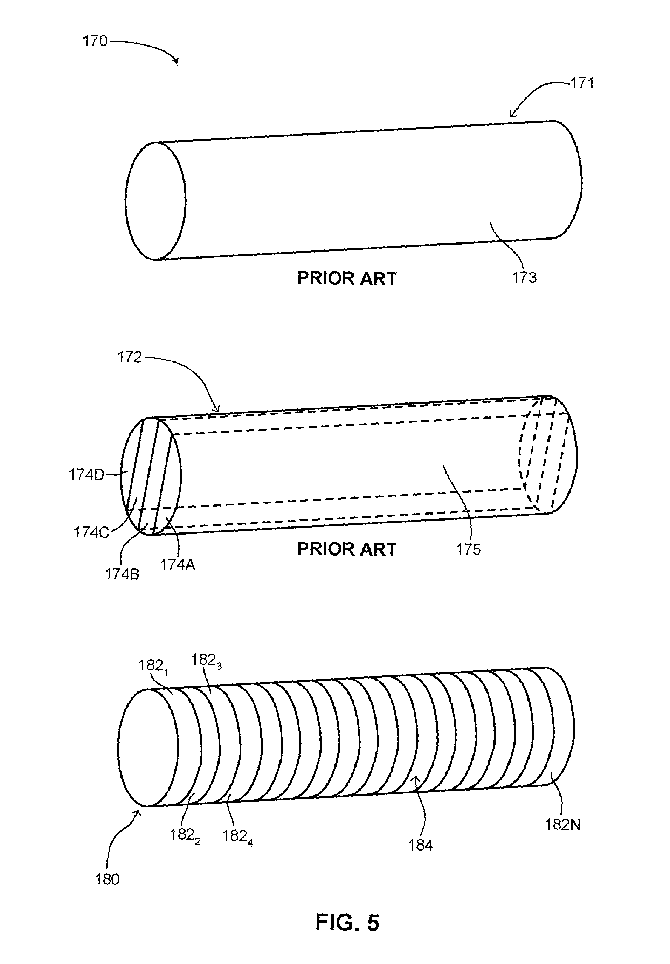

Reference is now made to Figure 5 which is a schematic illustration of various capacitor configurations, generally referenced 170, constructed and operative in accordance with another embodiment of the disclosed technique. A first capacitor configuration 171 is shown which includes a single capacitor having a cylindrical shape and includes a single anode 173 and a single cathode (not shown). First capacitor configuration 171 forms part of the prior art. As discussed above, such a configuration does not meet the energy and voltage requirements for a subcutaneous ICD while also meeting the volume and shape constraints of a flexible tubular device.

A second capacitor configuration 172 is shown which includes a plurality of anodes 174A, 174B, 174C and 174D and a respective plurality of cathodes (not shown). Plurality of anodes 174-174D together forms a cylindrical shape 175. Plurality of anodes 174-174D is arranged widthwise as elongated anodes, similar to first capacitor configuration 171. Second capacitor configuration 172 also forms part of the prior art and also does not meet the energy and voltage requirements for a subcutaneous ICD while also meeting the volume and shape constraints of a flexible tubular device as mentioned above due to the increased distance between any point in one of the anodes to any point in its respective cathode. The increased distance causes an increase in ESR thereby not enabling sufficient energy and voltage to be stored in such a capacitor configuration while nonetheless keeping its overall size and volume to a minimum.

A third capacitor configuration 180 is shown which includes a plurality of anodes 182₁, 182₂, 182₃, 182₄ and 182_{N} and a respective plurality of cathodes (not shown). Plurality of anodes 182₁-182_{N} together forms a cylindrical shape 184. Plurality of anodes 182₁-182_{N} is arranged lengthwise, thus forming a stack of flat, disc-shaped capacitors. As the inventors have discovered, such a configuration minimizes the ESR in a capacitor configuration, thereby enabling the requisite energy and voltage (approximately 70-80 joules and 1200 volts) for a subcutaneous ICD. In addition, such a capacitor configuration meets the size, shape and volume constraints of a flexible, tubular device to be implanted subcutaneously and function as an ICD, such as the ISSD as mentioned above. It is noted that plurality of anodes 182₁-182_{N} can also be a plurality of capacitors (not shown).

As described above, in the case of a subcutaneous ICD where significant energy and voltage is required, the cylindrical anode of first capacitor configuration 171 as well as the split elongated anode structure of second capacitor configuration 172 do not meet the requirements of energy and voltage while also meeting the spatial requirements of flexible, tubular structure. In general, long dual anode or multiple anode wet tantalum capacitors in cylindrical shape (such as shown in first and second capacitor configurations 171 and 172) are technically challenging and usually do not meet the high energy requirements for a subcutaneous ICD in a small volume, as is desired in a subcutaneous ICD. The increase in ESR in such capacitor configurations causes a significant reduction in energy and voltage per volume as compared with third capacitor configuration 180. Whereas this reduction in energy and voltage per volume can be compensated for by elongating the anodes, the resultant anodes and capacitors will not meet the spatial requirements of a subcutaneous ICD to be implanted under the skin of a patient around his heart. Such anodes and capacitors will be either too thick to be implanted subcutaneously, bulging out of the skin of the patient and being uncomfortable as well as not aesthetically pleasing or too long to be implanted subcutaneously in a patient in a noninvasive manner.

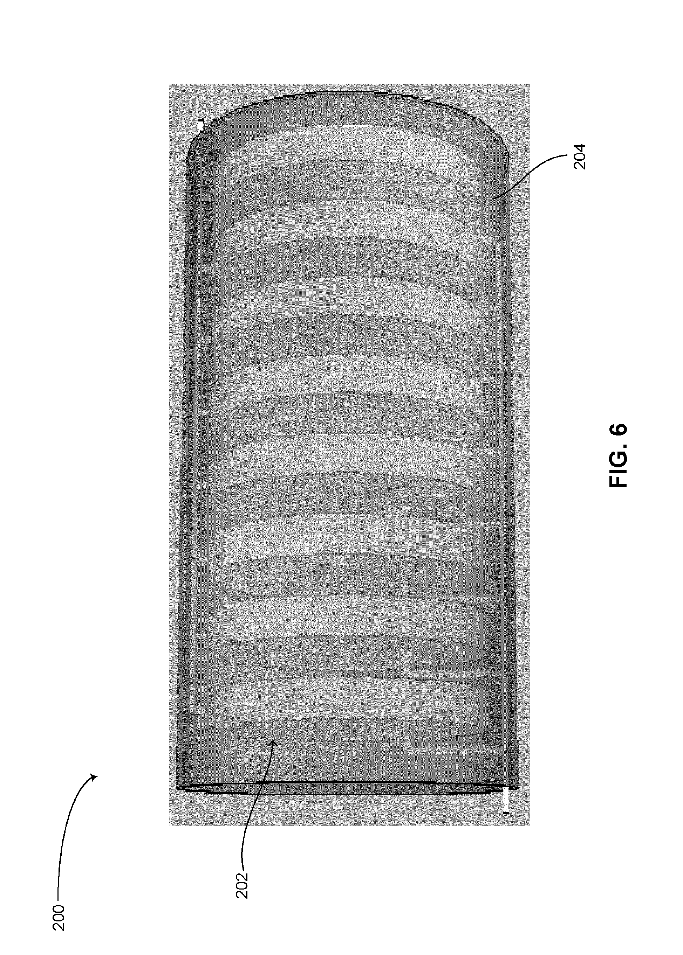

Reference is now made to Figure 6 which is a schematic illustration of the capacitor array of Figure 4 including a cover, generally referenced 200, constructed and operative in accordance with a further embodiment of the disclosed technique. As shown is a capacitor array 202, which is substantially similar to capacitor array 150 (Figure 4). Capacitor array 202 can be encapsulated in a cover 204. Cover 204 may be a cylindrical shell as well. Cover 204 can provide mechanical support to capacitor array 202 as well as protection.

Reference is now made to Figure 7 which is a schematic illustration of a high energy wet tantalum capacitor configuration for use in a flexible subcutaneous ICD, generally referenced 220, constructed an operative in accordance with another embodiment of the disclosed technique. Shown in Figure 7 is a plurality of capacitor arrays 222A, 222B, 222C, 222D and 222E with respective covers (not labeled). Each one of capacitor arrays 222A-222E is substantially similar to capacitor array 200 (Figure 6). As shown, within each capacitor array, the capacitors (not labeled) are coupled in parallel. Each capacitor array is coupled to a neighboring capacitor array in series. As shown, capacitor array 222A is coupled in series with capacitor array 222B via an electrical line 224, capacitor array 222B is coupled in series with capacitor array 222C via an electrical line 226, capacitor array 222C is coupled in series with capacitor array 222D via an electrical line 228 and capacitor array 222D is coupled in series with capacitor array 222E via an electrical line 230. Each electrical line couples the last cathode (not labeled) of a given capacitor array to the first anode (not labeled) of a subsequent capacitor array. By coupling a plurality of capacitor arrays in series as shown in Figure 7, a larger capacitor which meets the energy and voltage level requirements of a subcutaneous ICD can be constructed while also satisfying the spatial configuration constraints of a flexible, tubular device. The structure of high energy wet tantalum capacitor configuration 220 enables the integration of a plurality of capacitors into a flexible, tubular shaped device which can be incorporated into a subcutaneous ICD such as the ISSD of NewPace Ltd. As mentioned above, the capacitor configuration shown has a circular cross-section, although it could also have an elliptical cross-section as well. The capacitor configuration shown in Figure 7 includes five capacitor arrays coupled in series as an example. Each of capacitor arrays 222A-222E may be able to produce 14 joules of energy and support a voltage of 235 volts. Thus in total, high energy wet tantalum capacitor configuration 220 is capable of delivering about 70 joules and 1175 volts. As is obvious to the worker skilled in the art, the specific number of capacitor arrays as well as the number of capacitor coupled in parallel per array is a design choice depending on the energy and voltage requirements of a given subcutaneous device.

Embodiments of the present invention have been described with particular reference to the examples illustrated. While specific examples are shown in the drawings and are herein described in detail, it should be understood, however, that the drawings and detailed description are not intended to limit the invention to the particular form disclosed. It will be appreciated that variations and modifications may be made to the examples described within the scope of the present invention.

It will be appreciated by persons skilled in the art that the disclosed technique is not limited to what has been particularly shown and described hereinabove. Rather the scope of the disclosed technique is defined only by the claims, which follow.

## Claims

1. Capacitor configuration (100) for use in a subcutaneous implantable cardioverter-defibrillator (ICD) having a tubular shape, **characterized by**:
said capacitor configuration comprising a first plurality of high volumetric efficiency capacitors (104₁-104_{N}), each one of said first plurality of high volumetric efficiency capacitors having a disc-shape, said first plurality of high volumetric efficiency capacitors being coupled in parallel (110A,110B) and arranged in a stack as a first array of capacitors (102₁), thereby forming a cylindrically-shaped capacitor (150).

2. The capacitor configuration according to claim 1, wherein said first plurality of high volumetric efficiency capacitors are wet tantalum capacitors.

3. The capacitor configuration according to claim 1 or 2, wherein said disc-shape has a circular cross-section.

4. The capacitor configuration according to claim 1 or 2, wherein said disc-shape has an elliptical cross-section.

5. The capacitor configuration according to any one of the preceding claims, wherein said first array of capacitors arranged in said stack minimizes equivalent series resistance (ESR) in said cylindrically-shaped capacitor.

6. The capacitor configuration according to any one of the preceding claims, wherein said first array of capacitors enables high energy and high voltage storage.

7. The capacitor configuration according to any one of the preceding claims, further comprising a second plurality of high volumetric efficiency capacitors (108₁-108_{N}), said second plurality of high volumetric efficiency capacitors being coupled in parallel (112A,112B) and arranged in a stack as a second array of capacitors (102₂), wherein said first array of capacitors is coupled in series (116₁) with said second array of capacitors.

8. The capacitor configuration according to claim 7, wherein said first array of capacitors and said second array of capacitors together enable high energy and high voltage storage.

9. The capacitor configuration according to claim 8, wherein said high energy is at least 50 joules and said high voltage is at least 1000 volts.

10. The capacitor configuration according to any one of the preceding claims, further comprising a cover (204) for said first array of capacitors.

11. The capacitor configuration according to claim 10, wherein said cover provides at least one of mechanical support and protection to said first array of capacitors.

12. The capacitor configuration according to claim 7, wherein said first array of capacitors and said second array of capacitors enable flexibility in said subcutaneous ICD having said tubular shape.
